# EUROPEAN PATENT APPLICATION

(11) **EP 4 282 959 A1**
(43) Date of publication of application: **29.11.2023**
(21) Application number: 22767531.1
(22) Date of filing: 10.03.2022
(51) Int. Cl.: C12N 9/10, C12N 15/77, C12P 13/08, C12P 13/06, C12P 13/12

(54) **NOVEL CITRATE SYNTHASE VARIANT AND METHOD FOR PRODUCING L-AMINO ACIDS USING SAME**

(30) Priority: 10.03.2021 KR 20210031641
(71) Applicant: CJ Cheiljedang Corporation, Seoul 04560 (KR)
(72) Inventor: CHANG, Jin Sook, Seoul 04560 (KR); KIM, Ju-yeon, Seoul 04560 (KR); KIM, Seon Hye, Seoul 04560 (KR); CHOI, Sun Hyoung, Seoul 04560 (KR); YOON, Byoung Hoon, Seoul 04560 (KR); KIM, Hyung Joon, Seoul 04560 (KR); CHO, Seung Hyun, Seoul 04560 (KR); LEE, Jaemin, Seoul 04560 (KR); KIM, Seo-Yun, Seoul 04560 (KR); LEE, Imsang, Seoul 04560 (KR)
(74) Representative: Weickmann & Weickmann PartmbB
(86) International application number: PCT/KR2022/003359
(87) International publication number: WO 2022/191635

(57) **Abstract**

The present disclosure relates to a novel citrate synthase variant, a microorganism comprising the variant, and a method for producing L-amino acids using the microorganism.

## Description

### [Technical Field]

The present disclosure relates to a novel citrate synthase variant, a microorganism including the variant, and a method for producing L-amino acids using the microorganism.

### [Background Art]

In order to produce L-amino acids and other beneficial substances, various researches have been carried out to develop microorganisms with high-efficiency production and technologies for fermentation processes. For example, target-specific approaches, such as a method of increasing expression of a gene encoding an enzyme involved in L-valine biosynthesis or a method of removing a gene unnecessary for biosynthesis, have been widely used (US 8465962 B2, KR 10-2153534 B1).

Meanwhile, citrate synthase (CS) is an enzyme that produces citrate by catalyzing the condensation of acetyl-CoA and oxaloacetate, which are produced during glycolysis of a microorganism, and it is also an important enzyme for determining carbon-flow into the TCA pathway.

The phenotypic changes in L-lysine-producing strains due to the deletion of gltA gene encoding citrate synthase were reported previously in a literature (Ooyen et al., Biotechnol. Bioeng., 109(8): 2070-2081, 2012). However, these strains with the deletion of gltA gene have disadvantages in that not only their growth is inhibited but also their sugar consumption rates are significantly reduced thus resulting in low lysine production per unit time. Accordingly, further research is still needed that takes into account both an effective increase in L-amino acid productivity and the growth of the strains.

### [Disclosure]

### [Technical Problem]

As a result of intensive efforts to produce L-amino acids in high yield, the present inventors have completed this application by confirming that a novel citrate synthase variant increases L-amino acid-producing ability.

### [Technical Solution]

An object of the present disclosure is to provide a citrate synthase variant in which lysine, which is the amino acid corresponding to position 415 of the amino acid sequence of SEQ ID NO: 1, is substituted with histidine.

Another object of the present disclosure is to provide a polynucleotide encoding the variant of the present disclosure.

Still another object of the present disclosure is to provide a microorganism of the genus *Corynebacterium,* including the variant of the present disclosure or a polynucleotide encoding the variant.

Still another object of the present disclosure is to provide a method for producing L-amino acids using the microorganism of the present disclosure.

Still another object of the present disclosure is to provide a composition for producing L-amino acids, including the microorganism of the present disclosure; a medium on which the microorganism of the present disclosure is grown; or a combination thereof.

### [Advantageous Effects]

When the citrate synthase variant of the present disclosure is used, L-amino acids can be produced with high yield.

### [Detailed Description of Preferred Embodiments]

Hereinafter, the present disclosure will be described in detail. Meanwhile, each description and embodiment disclosed herein can be applied to other descriptions and embodiments with respect to common features. That is, all combinations of various elements disclosed herein fall within the scope of the present disclosure. Further, the scope of the present disclosure is not limited by the specific description described below. In addition, a number of papers and patent documents have been referenced and cited throughout the present specification. The content of the cited papers and patent documents is incorporated herein by reference in their entirety, and the level of technical field to which the present invention belongs and the contents of the present invention will be described more clearly.

One aspect of the present disclosure provides a citrate synthase variant in which lysine, which is the amino acid corresponding to position 415 of the amino acid sequence of SEQ ID NO: 1, is substituted with histidine.

The variant of the present disclosure may be a variant, in which the amino acid corresponding to position 415 based on the amino acid sequence of SEQ ID NO: 1 in the amino acid sequence represented by SEQ ID NO: 1 is histidine, and which has a homology or identity of at least 70%, 75%, 80%, 85%, 90%, 95%, 96%, 97%, 98%, 99%, 99.5%, 99.7% or 99.9% or more to the amino acid sequence represented by SEQ ID NO: 1. For example, the variant of the present disclosure may be a variant, in which the amino acid corresponding to position 415 based on the amino acid sequence of SEQ ID NO: 1 in the amino acid sequence represented by SEQ ID NO: 1 is histidine, and may have or include an amino acid sequence having a homology or identity of at least 70%, 75%, 80%, 85%, 90%, 95%, 96%, 97%, 98%, 99%, 99.5%, 99.7% or 99.9% or more to the amino acid sequence represented by SEQ ID NO: 1, or may consist of or consist essentially of the amino acid sequence. Additionally, it is apparent that any variant having an amino acid sequence, in which part of the sequence is deleted, modified, substituted, conservatively substituted or added, may also fall within the scope of the present disclosure as long as the amino acid sequence has such a homology or identity and exhibits an efficacy corresponding to that of the variant of the present disclosure.

For example, it may be a case where the N-terminus, C-terminus and/or inside of the amino acid sequence is added or deleted with a sequence that does not alter the function of the variant of the present disclosure, a naturally occurring mutation, a silent mutation, or a conservative substitution.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of the protein or polypeptide.

As used herein, the term "variant" refers to a polypeptide having one or more amino acids different from the amino acid sequence of the variant before mutation by conservative substitutions and/or modifications such that the functions and properties of the protein are retained. Such variants may generally be identified by modifying one or more of the above amino acid sequences of the polypeptide and evaluating the properties of the modified polypeptide. That is, the ability of the variants may be enhanced, unchanged or reduced relative to a polypeptide before mutation. Additionally, some variants may include those in which one or more regions, such as an N-terminal leader sequence or transmembrane domain, have been removed. Further, other variants may include those in which a region has been removed from the N- and/or C-terminal of a mature protein. The term "variant" may be used interchangeably with terms such as modification, modified polypeptide, modified protein, mutant, mutein, divergent, *etc.,* as long as the terms are used to indicate mutation. For the purpose of the present disclosure, the variant may be a variant in which lysine (Lys, K), which is the amino acid corresponding to position 415 of the amino acid sequence of SEQ ID NO: 1, is substituted with histidine (His, H).

Additionally, the variants may also include deletion or addition of amino acids that have minimal influence on the properties and secondary structure of a polypeptide. For example, the variants may be conjugated with a signal (or leader) sequence at the N-terminal involved in the translocation of proteins co-translationally or post-translationally. Further, the variants may also be conjugated with another sequence or linker to identify, purify, or synthesize the polypeptide.

As used herein, the term 'homology' or 'identity' refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ET AL.](1988) SIAM J Applied Math 48: 1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48: 443 as disclosed in Smith and Waterman, Adv. Appl. Math (1981) 2:482. In summary, the GAP program defines the homology, similarity, or identity as the value obtained by dividing the number of similarly aligned symbols (*i.e.,* nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), *etc.,* but is not limited thereto, and sequence alignment programs, such as pairwise sequence comparison algorithms, *etc.,* known in the art may be appropriately used.

As used herein, the term "citrate synthase" refers to an enzyme that produces citrate by catalyzing the condensation of acetyl-CoA and oxaloacetate, which are produced during the glycolysis of a microorganism. Additionally, the citrate synthase catalyzes the condensation reaction of a two-carbon acetate residue from acetyl-CoA and a molecule of 4-carbon oxaloacetate to form a 6-carbon oxaloacetate. The term "citrate synthase" may be used interchangeably with "enzyme for synthesizing citrate", "CS", "GltA protein", or "GltA". In the present disclosure, the sequence of the GltA can be obtained from NCBI's GenBank, a known database. In addition, the GltA may be a polypeptide having citrate synthase activity encoded by the *gltA* gene, but is not limited thereto.

The variant of the present disclosure may have an activity of increasing L-amino acid-producing ability compared to a wild-type polypeptide.

The variant of the present disclosure may have a sequence identity of 80% or more with the amino acid sequence of SEQ ID NO: 1.

Additionally, the variant of the present disclosure may include a polypeptide represented by the amino acid sequence of SEQ ID NO: 3. The amino acid sequence of SEQ ID NO: 3 may be an amino acid sequence in which lysine corresponding to position 415 in the amino acid sequence at position 362 to position 415 from the N-terminus of the amino acid sequence of SEQ ID NO: 1 is substituted with histidine.

The variant of the present disclosure may include the amino acid sequence of General Formula 1 below: 
X1N HGGDATX2FMN KVKNKEDGVR LMGFGHRVYK NYDPRAAIVK
ETAHEILEHL GGDDLLDLAI KLEEIALADD X3FISRKLYPN VDFYTGLIYR
AMGFPTDFFT VLFAIGRLPG WIAHYREQLG AAGNH (SEQ ID NO: 51);
wherein in General Formula 1 above,
X₁ is asparagine or serine;
X₂ is alanine or glutamic acid; and
X₃ is tyrosine or cysteine.

The variant of the present disclosure may have a sequence identity of 90% or more with the amino acid sequence of SEQ ID NO: 8, 10, or 12. Additionally, the variant of the present disclosure may include, consist of, or consist essentially of an amino acid sequence having a sequence identity of 90% or more with the amino acid sequence of SEQ ID NO: 8, 10, or 12. For example, the variant of the present disclosure may have a sequence identity of 90%, 91%, 92%, 93%, 94%, 95%, 96%, 97%, 98%, 99%, 99.5%, or 99.7% with the amino acid sequence of SEQ ID NO: 8, 10, or 12, may include an amino acid sequence having the sequence identity, or may consist of or essentially consist of an amino acid sequence having the sequence identity.

Another aspect of the present disclosure provides a polynucleotide encoding the variant of the present disclosure.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the variant.

In the polynucleotide of the present disclosure, the nucleotides corresponding to positions 1243 to 1245 based on the nucleic acid sequence of SEQ ID NO: 2 are CAC, and any polynucleotide represented by a nucleic acid sequence having a homology or identity of at least 70%, 75%, 80%, 85%, 90%, 95%, 96%, 97%, 98%, 99%, 99.5%, 99.7%, or 99.9% or more, and less than 100% with the nucleic acid sequence represented by SEQ ID NO: 2 may be included. Additionally, it is apparent that any polynucleotide represented by a nucleic acid sequence, in which part of the sequence is deleted, modified, substituted, conservatively substituted or added, may also fall within the scope of the present disclosure as long as the sequence has such a homology or identity and encodes a polypeptide or protein exhibiting an efficacy corresponding to that of the variant of the present disclosure.

The polynucleotide of the present disclosure may undergo various modifications in the coding region within the scope that does not change the amino acid sequence of the variant of the present disclosure, due to codon degeneracy or in consideration of the codons preferred in an organism in which the variant of the present disclosure is to be expressed. Here, in the sequence having the homology or identity, the codon encoding the amino acid corresponding to position 415 of SEQ ID NO: 1 may be one of the codons encoding histidine.

Further, the polynucleotide of the present disclosure may include a probe that may be prepared from a known gene sequence, for example, any sequence which can hybridize with a sequence complementary to all or part of the polynucleotide sequence of the present disclosure under stringent conditions without limitation. The "stringent conditions" refers to conditions under which specific hybridization between polynucleotides is allowed. Such conditions are specifically described in the literature (J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity of 70% or more, 75% or more, 80% or more, 85% or more, 90% or more, 95% or more, 96% or more, 97% or more, 98% or more, or 99% or more are hybridized with each other and polynucleotides having a homology or identity lower than the above homologies or identities are not hybridized with each other, or washing conditions of Southern hybridization, that is, washing once, specifically, twice or three times at a salt concentration and a temperature corresponding to 60°C, 1 × SSC, 0.1% SDS, specifically, 60°C, 0.1× SSC, 0.1% SDS, and more specifically 68°C, 0.1 × SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present disclosure may include isolated nucleotide fragments complementary to the entire sequence as well as nucleic acid sequences substantially similar thereto.

Specifically, polynucleotides having a homology or identity with the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g.,* Sambrook et al.).

In one example, the polynucleotide of the present disclosure may include a polynucleotide represented by the nucleic acid sequence at positions 1084 to 1245 based on the nucleic acid sequence of SEQ ID NO: 9, 11 or 13; or a polynucleotide represented by the nucleic acid sequence of SEQ ID NO: 9, 11, 13 or 15.

In the polynucleotide of the present disclosure, the variant is as described in the other aspects above.

Still another aspect of the present disclosure provides a vector containing the polynucleotide of the present disclosure. The vector may be an expression vector for expressing the polynucleotide in a host cell, but is not limited thereto.

The vector of the present disclosure may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL and pET, *etc.* may be used. Specifically, pDZ, pDC, pDCM2, pACYC177, pACYC184, pCL, pECCG117(Biotechnology letters vol 13, No. 10, p. 721-726(1991), Korean Patent No. 10-1992-0007401), pUC19, pBR322, pMW118, pCC1 BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target variant of the present disclosure.

In the vector of the present disclosure, the variant and polynucleotide are as described in the other aspects above.

Yet another aspect of the present disclosure provides a microorganism of the genus *Corynebacterium,* including the variant of the present disclosure or the polynucleotide of the present disclosure.

The microorganism of the present disclosure may include the variant of the present disclosure, a polynucleotide encoding the variant, or a vector containing the polynucleotide of the present disclosure.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product.

The microorganism of the present disclosure may be a microorganism including any one or more of the variant of the present disclosure, the polynucleotide of the present disclosure, and the vector containing the polynucleotide of the present disclosure; a microorganism modified to express the variant of the present disclosure or the polynucleotide of the present disclosure; a microorganism (*e.g.,* a recombinant strain) expressing the variant of the present disclosure or the polynucleotide of the present disclosure; or a microorganism (*e.g.,* a recombinant strain) having the variant activity of the present disclosure, but is not limited thereto.

The microorganism of the present disclosure may have an L-amino acid-producing ability. Specifically, in the microorganism of the present disclosure, the L-amino acid-producing ability may be an L-valine or O-acetyl-L-homoserine-producing ability.

The microorganism of the present disclosure may be a microorganism that naturally has a GltA or L-amino acid-producing ability, or a microorganism that has been introduced with the variant of the present disclosure or the polynucleotide encoding the same (or the vector containing the polynucleotide) to a parent strain that does not naturally have a GltA or L-amino acid-producing ability, and/or a microorganism that has been given a GltA or L-amino acid-producing ability, but is not limited thereto.

In one example, the microorganism of the present disclosure is a cell or microorganism transformed with the polynucleotide of the present disclosure or a vector containing the polynucleotide of the present disclosure to express the variant of the present disclosure, and for purposes of the present disclosure, the microorganism of the present disclosure may include all microorganisms capable of producing L-amino acids, including the variant of the present disclosure. For example, the strain of the present disclosure may be a recombinant strain whose L-amino acid-producing ability is increased by introducing the polynucleotide encoding the variant of the present disclosure into a natural wild-type microorganism or a microorganism producing L-amino acids. The recombinant strain with an increased L-amino acid-producing ability may be a microorganism having an increased L-amino acid-producing ability compared to a natural wild-type microorganism or a non-modified microorganism of citrate synthase (*i.e.,* a microorganism expressing a wild-type protein (SEQ ID NO: 1) or a microorganism not expressing the variant of the present disclosure), but is not limited thereto. For example, the non-modified microorganism of citrate synthase, which is the target strain for comparing the increase in the L-amino acid-producing ability, may be ATCC14067 strain, ATCC13032 strain, ATCC13869 strain, *Corynebacterium glutamicum* CJ7V strain, *Corynebacterium glutamicum* CJ8V strain, or CA08-0072 strain, but is not limited thereto.

In one example, the recombinant strain having an increased production ability may have an increased L-amino acid-producing ability by about 1% or more, 5% or more, 7% or more, about 10% or more, about 20% or more, or about 30% or more (the upper limit is not particularly limited, for example, about 200% or less, about 150% or less, about 100% or less, about 50% or less, about 45% or less, about 40% or less, or about 30% or less) as compared to the L-amino acid-producing ability of a parent strain before modification or a non-modified microorganism, but is not limited thereto, as long as it has an increased + value compared to the production ability of a parent strain before modification or a non-modified microorganism. In another example, the recombinant strain having an increased production ability may have an increased L-amino acid-producing ability by about 1.01 times or more, about 1.05 times or more, about 1.07 times or more, about 1.1 times or more, about 1.2 times or more, or about 1.3 times or more (the upper limit is not particularly limited, for example, about 10 times or less, about 5 times or less, about 3 times or less, or about 2 times or less) as compared to that of a parent strain before modification or a non-modified microorganism.

As used herein, the term "about" refers to a range which includes all of ±0.5, ±0.4, ±0.3, ±0.2, ±0.1, *etc.,* and includes all of the values that are equivalent or similar to those following the values, but the range is not limited thereto.

As used herein, the term "non-modified microorganism" does not exclude a strain containing a mutation that may occur naturally in a microorganism, and may refer to a wild-type strain or natural-type strain itself, or a strain before the trait is altered due to genetic modification caused by natural or artificial factors. For example, the non-modified microorganism may refer to a strain into which the protein variant described herein is not introduced, or a strain before the introduction thereof. The "non-modified microorganism" may be used interchangeably with "strain before modification", "microorganism before modification", "non-mutant strain", "non-modified strain", "non-mutant microorganism" or "reference microorganism".

In another example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

The microorganism of the present disclosure may be a microorganism in which the NCgl2335 protein is further weakened. Additionally, the microorganism of the present disclosure may be a microorganism in which the activity of protein selected from the group consisting of acetolactate synthase isozyme 1 small subunit (IlvN) or L-methionine/branched-chain amino acid exporter (YjeH) is further enhanced.

Specifically, the microorganism producing L-valine of the present disclosure may be a microorganism in which the activity of IlvN is further enhanced and/or the NCgl2335 protein is further weakened. Additionally, the microorganism producing O-acetyl-L-homoserine of the present disclosure may be a microorganism in which the activity of L-methionine/branched-chain amino acid exporter (YjeH) is further enhanced.

As used herein, the term "weakening" of the activity of a polypeptide is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity and/or concentration (expression level) is decreased compared to a natural strain due to the inhibition of expression of the gene of the polynucleotide encoding the polypeptide, or the inhibition of translation into the polypeptide, *etc.;* a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity can be performed by any method known in the art, but the method is not limited thereto, and can be achieved by applying various methods well known in the art (*e.g.,* Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide activity of the present disclosure may be:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e.g.,* deletion/substitution/addition of one or more amino acids on the amino acid sequence);
4) modifying the gene sequence encoding the polypeptide such that the polypeptide activity is removed or weakened (*e.g.,* deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene transcript encoding the polypeptide;
6) introducing an antisense oligonucleotide (*e.g.,* antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide; or
9) a combination of two or more selected from the 1) to 8) above, but is not particularly limited thereto.

For example,
The 1) deleting a part or all of the gene encoding the polypeptide may be deleting all of the polynucleotide encoding the endogenous target polypeptide within the chromosome, or replacing the polynucleotide with a polynucleotide having a partially deleted nucleotide, or with a marker gene.
The 2) modifying the expression regulatory region (expression regulatory sequence) may be inducing a modification on the expression regulatory region (expression regulatory sequence) through deletion, insertion, non-conservative substitution or conservative substitution, or a combination thereof; or replacing the sequence with a sequence having a weaker activity. The expression regulatory region may include a promoter, an operator sequence, a sequence encoding a ribosome-binding site, and a sequence for regulating the termination of transcription and translation, but is not limited thereto.
The 3) and 4) modifying the amino acid sequence or the polynucleotide sequence may be inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.
The 5) modifying the nucleotide sequence encoding the initiation codon or 5'-UTR of the gene transcript encoding the polypeptide may be , for example, substituting the nucleotide sequence with a nucleotide sequence encoding another initiation codon having a lower polypeptide expression rate than the endogenous initiation codon, but is not limited thereto.
The 6) introducing an antisense oligonucleotide (*e.g.,* antisense RNA), which binds complementary to the gene transcript encoding the polypeptide, can be found in the literature [Weintraub, H. et al., Antisense-RNA as a molecular tool for genetic analysis, Reviews - Trends in Genetics, Vol. 1(1) 1986].
The 7) adding a sequence complementary to the Shine-Dalgarno (SD) sequence on the front end of the SD sequence of the gene encoding the polypeptide to form a secondary structure, thereby inhibiting the ribosome attachment may be inhibiting mRNA translation or reducing the speed thereof.
Further, the 8) reverse transcription engineering (RTE), which adds a promoter, which is to be reversely transcribed, on the 3' terminus of the open reading frame (ORF) of the gene sequence encoding the polypeptide, may be forming an antisense nucleotide complementary to the gene transcript encoding the polypeptide to weaken the activity.

As used herein, the term "enhancement" of the activity of a polypeptide means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

The enhancement of the activity of the polypeptide can be applied by various methods well known in the art, and the method is not limited as long as it can enhance the activity of the target polypeptide compared to that of a microorganism before modification. Specifically, genetic engineering and/or protein engineering well known to those skilled in the art, which is a common method of molecular biology, may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

Specifically, the enhancement of the activity of the polypeptide of the present disclosure may be:
1) increasing the intracellular copy number of a polynucleotide encoding the polypeptide;
2) replacing the expression regulatory region of a gene encoding the polypeptide on the chromosome with a sequence having a stronger activity;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g.,* modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

More specifically,
The 1) increasing the intracellular copy number of a polynucleotide encoding the polypeptide may be introducing a vector, which is operably linked to the polynucleotide encoding the polypeptide and is able to replicate and function regardless of a host cell, into the host cell. Alternatively, it may be introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.
The 2) replacing the expression regulatory region (or expression regulatory sequence) of a gene encoding the polypeptide on the chromosome with a sequence having a strong activity may be, for example, inducing a modification on the sequence through deletion, insertion, non-conservative or conservative substitution, or a combination thereof to further enhance the activity of the expression regulatory region, or replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, it may be replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) modifying the nucleotide sequence encoding the initiation codon or 5'-UTR of the gene transcript encoding the polypeptide may, for example, substituting the nucleotide sequence with a nucleotide sequence encoding another initiation codon having a higher expression rate of the polypeptide compared to the endogenous initiation codon, but is not limited thereto.

The 4) and 5) modifying the amino acid sequence or the polynucleotide sequence may be inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) introducing a foreign polynucleotide exhibiting the activity of the polypeptide may be introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) codon-optimization of the polynucleotide encoding the polypeptide may be codon-optimization of an endogenous polynucleotide to increase the transcription or translation within a host cell, or optimizing the codons thereof such that the optimized transcription and translation of the foreign polynucleotide can be achieved within the host cell.

Further, the 8) analyzing the tertiary structure of the polypeptide and thereby selecting and modifying the exposed site, or chemically modifying the same may be, for example, comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration (expression level) of the corresponding polypeptide is increased relative to the activity or concentration (expression level) of the polypeptide expressed in a wild-type strain or a microorganism before modification, or that the amount of product produced from the polypeptide is increased, but is not limited thereto.

The modification of a part or all of the polynucleotide in the microorganism of the present disclosure (*e.g.,* modification for encoding the protein variant described above) may be achieved by (a) homologous recombination using a vector for chromosomal insertion in the microorganism or genome editing using an engineered nuclease (*e.g.,* CRISPR-Cas9), and/or (b) may be induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

More specifically, the microorganism producing L-valine of the present disclosure may be a microorganism including a polypeptide represented by the amino acid sequence of SEQ ID NO: 27 and/or a polynucleotide represented by the nucleotide sequence of SEQ ID NO: 28. Further, the microorganism producing O-acetyl-L-homoserine of the present disclosure may be a microorganism including a polypeptide represented by the amino acid sequence of SEQ ID NO: 47 and/or a polynucleotide represented by the nucleotide sequence of SEQ ID NO: 48; and may be a microorganism including a mutation selected from the group consisting of inactivation of a polypeptide represented by the amino acid sequence of SEQ ID NO: 37 and/or deletion of a polynucleotide represented by the nucleotide sequence of SEQ ID NO: 38.

In the microorganisms of the present disclosure, the variant, polynucleotide, *etc.* are as described in the other aspects above.

Even another aspect of the present disclosure provides a method for producing L-amino acids, including: culturing a microorganism of the genus *Corynebacterium,* which includes the variant of the present disclosure or the polynucleotide of the present disclosure, in a medium.

The method for producing L-amino acids of the present disclosure may include culturing a *Corynebacterium glutamicum* strain, which includes the variant of the present disclosure, the polynucleotide of the present disclosure, or the vector of the present disclosure, in a medium.

Further, in the method for producing L-amino acids of the present disclosure, the L-amino acid may be L-valine, O-acetyl-L-homoserine, or L-methionine.

As used herein, the term "cultivation" means that the microorganism of the genus *Corynebacterium* of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the genus *Corynebacterium* of the present disclosure as a main ingredient, and it supplies nutrient materials and growth factors, along with water that is essential for survival and growth. Specifically, the medium and other culture conditions used for culturing the microorganism of the genus *Corynebacterium* of the present disclosure may be any medium used for conventional cultivation of microorganisms without any particular limitation. However, the microorganism of the genus *Corynebacterium* of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

Specifically, the culture medium for the microorganism of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of *Corynebacterium glutamicum* strain of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may carried be out for about 10 to 160 hours, but the cultivation is not limited thereto.

The L-amino acids produced by the cultivation of the present disclosure may be released into the medium or remain in the cells.

The method for producing L-amino acids of the present disclosure may further include a step of preparing the microorganism of the genus *Corynebacterium* of the present disclosure, a step of preparing a medium for culturing the microorganism, or a combination thereof (regardless of the order, in any order), for example, prior to the culturing step.

The method for producing L-amino acids of the present disclosure may further include a step of recovering L-amino acids from the culture medium (medium on which the culture was grown) or the microorganism of the genus *Corynebacterium* of the present disclosure. The recovering step may be further included after the culturing step.

In the recovering step, the desired L-amino acids may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired L-amino acids can be recovered from the medium or the microorganisms using suitable methods known in the art.

Further, the method for producing L-amino acids of the present disclosure may further include a purification step, which may be performed using an appropriate method known in the art. In one example, when the method for producing L-amino acids of the present disclosure includes both a recovering step and a purification step, the recovering step and the purification step may be performed continuously or intermittently regardless of the order or simultaneously, or may be integrated into one step, but the method is not limited thereto.

Moreover, the method for producing L-methionine of the present disclosure may further include a step of converting the O-acetyl-L-homoserine into L-methionine. In the method for producing L-methionine of the present disclosure, the conversion step may be further included after the culturing step or the recovering step. The conversion step may be performed using a suitable method known in the art (US 8426171 B2). In one embodiment, the method for producing L-methionine of the present disclosure may include a step of producing L-methionine by contacting O-acetyl-L-homoserine and methyl mercaptan with O-acetylhomoserine sulfhydrylase, cystathionine gamma-synthase, or O-succinyl homoserine sulfhydrylase.

In the method of the present disclosure, the variant, polynucleotide, vector, microorganism, *etc.,* are as described in the other aspects above.

Further another aspect of the present disclosure provides a composition for producing L-amino acids, including: a microorganism of the genus *Corynebacterium,* which includes the variant of the present disclosure, the polynucleotide encoding the variant of the present disclosure, or the vector containing the polynucleotide of the present disclosure; a medium on which the microorganism is grown; or a combination thereof.

The composition of the present disclosure may further include any suitable excipient commonly used in compositions for producing amino acids, and such excipients include, for example, preservatives, wetting agents, dispersing agents, suspending agents, buffers, stabilizers, or isotonic agents, *etc.,* but are not limited thereto.

In the composition for producing L-amino acids of the present disclosure, the L-amino acid may be L-valine, O-acetyl-L-homoserine, or L-methionine.

In the composition of the present disclosure, the variant, polynucleotide, vector, strain, medium, *etc.,* are as described in the other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Construction of Citrate Synthase (GltA) Variant Vector

The present inventors have discovered the 415^{th} amino acid residue of GltA as an acetyl-CoA binding site, and predicted that when the amino acid was substituted with another amino acid, citrate synthase activity would be weakened as the Km value of acetyl-CoA increased.

Accordingly, a vector, in which lysine, the 415^{th} amino acid of GltA, is substituted with another amino acid, was constructed. Specifically, a vector containing mutations, in which lysine, the 415^{th} amino acid of GltA, is substituted with histidine (K415H), tryptophan (K415W), and glycine (K415G), was constructed.

PCR was performed using primer pairs of SEQ ID NOS: 15 and 17, and SEQ ID NOS: 16 and 18, primer pairs of SEQ ID NOS: 15 and 20, and SEQ ID NOS: 18 and 19, and primer pairs of SEQ ID NOS: 15 and 22, and SEQ ID NOS: 18 and 21, based on the wild-type *Corynebacterium glutamicum* ATCC14067 gDNA as a template. Overlapping PCR was performed based on the mixture of the two fragments among six fragments obtained above as a template using the primer pair of SEQ ID NOS: 15 and 18 to obtain three fragments. The PCR was performed under conditions of denaturation at 94°C for 5 minutes, followed by 30 cycles of denaturation at 94°C for 30 seconds, annealing at 55°C for 30 seconds, and polymerization at 72°C for 1 minute and 30 seconds, and then polymerization at 72°C for 5 minutes. The pDCM2 vector (SEQ ID NO: 14, Korean Application Publication No. 10-2020-0136813) was treated with smal, and each of the three PCR products obtained above was subjected to fusion cloning. The fusion cloning was performed using an In-Fusion^{®} HD cloning kit (Clontech). The plasmids obtained as a result of the cloning above were named pDCM2-gltA(K415H), pDCM2-gltA(K415W), and pDCM2-gltA(K415G), respectively. The sequences of the primers used in this Example are shown in Table 1 below.

**[Table 1]**

| SEQ ID NO: | Primers | Sequences |
|---|---|---|
| 15 | Primer 1 | TCGAGCTCGGTACCC CCGTTCGTATGATCGGTTCCGCACAGGCC |
| 16 | Primer 2 | GTGCAGCAGGCAAC CAC ATCAACCGCCCACG |
| 17 | Primer 3 | CGTGGGCGGTTGAT GTG GTTGCCTGCTGCAC |
| 18 | Primer 4 | CTCTAGAGGATCCCC GCCGTAAGCAGCCTCTGGTGGAATGGTCAGC |
| 19 | Primer 5 | GTGCAGCAGGCAAC TGG ATCAACCGCCCACG |
| 20 | Primer 6 | CGTGGGCGGTTGAT CCA GTTGCCTGCTGCAC |
| 21 | Primer 7 | GTGCAGCAGGCAAC GGC ATCAACCGCCCACG |
| 22 | Primer 8 | CGTGGGCGGTTGAT GCC GTTGCCTGCTGCAC |

### Example 2: Introduction of GitA Variant into L-Valine-Producing Strains and Evaluation thereof

### 2-1. Construction of L-Valine Production-Based Strains and Evaluation thereof

One kind of mutation [ilvN(A42V); Biotechnology and Bioprocess Engineering, June 2014, Volume 19, Issue 3, pp 456-467] (SEQ ID NO: 27) was introduced into acetolactate synthase isozyme 1 small subunit (IlvN) of the wild-type *Corynebacterium glutamicum* ATCC14067 and ATCC13869 to construct strains having an enhanced L-valine-producing ability (KR 10-1947945 B1).

Specifically, PCR was performed using primer pairs of SEQ ID NOS: 29 and 31, and SEQ ID NOS: 30 and 32, based on the wild-type *Corynebacterium glutamicum* ATCC14067 gDNA as a template. Overlapping PCR was performed based on the mixture of the two fragments obtained above as a template using the primer pair of SEQ ID NOS: 29 and 32 to obtain three fragments. Here, the PCR was performed under conditions of denaturation at 94°C for 5 minutes, followed by 30 cycles of denaturation at 94°C for 30 seconds, annealing at 55°C for 30 seconds, and polymerization at 72°C for 1 minute and 30 seconds, and then polymerization at 72°C for 5 minutes. The pDCM2 vector was treated with smal, and the three PCR products obtained above were each subjected to fusion cloning. The plasmid obtained as a result of the cloning above was named pDCM2-ilvN(A42V). Thereafter, the pDCM2-ilvN(A42V) was transformed into the wild-type *Corynebacterium glutamicum* ATCC14067 and ATCC13869 strains to induce homologous recombination on the chromosome (van der Rest et al., Appl Microbiol Biotechnol 52:541-545, 1999). The strains introduced with the vector on the chromosome by recombination of homologous sequences were selected in a medium containing 25 mg/L of kanamycin. The gene fragments were amplified based on the selected *Corynebacterium glutamicum* transformants by PCR using a primer pair of SEQ ID NOS: 33 and 34, and the introduction of the mutation was confirmed by gene sequencing analysis. The recombinant strains were named *Corynebacterium glutamicum* CJ7V and CJ8V, respectively. The sequences of the primers used in this Example are shown in Table 2 below.

**[Table 2]**

| SEQ ID NO: | Name of Sequences | Sequences |
|---|---|---|
| 29 | Primer 11 | TCGAGCTCGGTACCCC CGCGTCACCAAAGCGGA |
| 30 | Primer 12 | GTCCCTCGTGTCTGTAAAGACCGAAACACT |
| 31 | Primer 13 | AGTGTTTCGGTCTTTACAGACACGAGGGAC |
| 32 | Primer 14 | CTCTAGAGGATCCCC TTAGATCTTGGCCGGAGCCA |
| 33 | Primer 15 | CCGCGTCACCAAAGCGGA |
| 34 | Primer 16 | TTAGATCTTGGCCGGAGCCA |

Thereafter, the experiment on fermentation titer was carried out based on the wild-type *Corynebacterium glutamicum* ATCC14067 and ATCC13869, and the CJ7V and CJ8V strains constructed above. Each strain was sub-cultured in a nutrient medium, and then seeded into a 250 ml corner-baffle flask containing 25 ml of a production medium and cultured at 30°C for 72 hours at 200 rpm under shaking. Thereafter, the concentration of L-valine was analyzed using HPLC, and the analyzed concentration of L-valine is shown in Table 3 below.

### <Nutrient Medium (pH 7.2)>

Glucose 10 g, Meat Extract 5 g, Polypeptone 10 g, Sodium Chloride 2.5 g, Yeast Extract 5 g, Agar 20 g, Urea 2 g (based on 1 L of distilled water)

### <Production Medium (pH 7.0)>

Glucose 100 g, (NH₄)₂SO4 40 g, Soy Protein 2.5 g, Corn Steep Solids 5 g, Urea 3 g, K₂HPO₄ 1 g, MgSO_{4·}7H₂O 0.5 g, Biotin 100 µg, Thiamine-HCl 1 mg, Calcium-Pantothenic Acid 2 mg, Nicotinamide 3 mg, CaCOs 30 g (based on 1 L of distilled water).

**[Table 3]**

| L-Valine-Producing Ability of L-Valine Production-Based Strains CJ7V and CJ8V | |
|---|---|
| Strains | L-Valine (g/L) |
| ATCC14067 | 1.5 |
| CJ7V(ilvN(A42V)) | 2.2 |
| ATCC13869 | 1.0 |
| CJ8V(ilvN(A42V)) | 1.9 |

As shown in the results, it was confirmed that the L-valine-producing ability was increased in the CJ7V and CJ8V strains introduced with ilvN(A42V) gene mutation as compared to the wild-type *Corynebacterium glutamicum* ATCC14067 and ATCC13869 strains.

### 2-2. Introduction of GitA-Weakened Variants (K415H, K415W, K415G) into L-Valine-Producing Strains and Evaluation thereof

The L-valine-producing ability was evaluated by introducing the GltA variants into the L-valine-producing strains. The pDCM2-gltA(K415H), pDCM2-gltA(K415W), and pDCM2-gltA(K415G) vectors constructed in Example 1 were transformed into each of the L-valine-producing strains CJ7V and CJ8V, and CA08-0072 (KCCM11201P, US 8465962 B2) by homologous recombination on the chromosome. The strains introduced with the vectors on the chromosome by recombination of homologous sequences were selected in a medium containing 25 mg/L of kanamycin.

Thereafter, the gene fragments were amplified based on the *Corynebacterium glutamicum* transformants, in which the secondary recombination is completed, by PCR using a primer pair of SEQ ID NOS: 23 and 24 (Table 4), and then the mutation-introduced strains were confirmed by gene sequencing analysis. The recombinant strains were named based on the *Corynebacterium glutamicum* as shown below, and the titer evaluation was carried out in the same manner as in Example 2-1. The results are shown in Table 5.

**[Table 4]**

| SEQ ID NO: | Name of Sequences | Sequences |
|---|---|---|
| 23 | Primer 9 | CCGTTCGTATGATCGGTTCCGCACAGGCC |
| 24 | Primer 10 | GCCGTAAGCAGCCTCTGGTGGAATGGTCAGC |

**[Table 5]**

| Strains | OD600 | L-Valine (g/L) |
|---|---|---|
| CJ7V | 77 | 2.2 |
| CJ7V:gltA(K415H) | 75 | 2.5 |
| CJ7V:gltA(K415W) | 47 | 1.2 |
| CJ7V:gltA(K415G) | 42 | 1.0 |
| CJ8V | 89 | 1.9 |
| CJ8V:gltA(K415H) | 89 | 2.1 |
| CJ8V:gltA(K415W) | 50 | 1.0 |
| CJ8V:gltA(K415G) | 49 | 1.0 |
| CA08-0072 | 62 | 2.6 |
| CA08-0072:gltA(K415H) | 60 | 2.9 |
| CA08-0072:gltA(K415W) | 35 | 1.8 |
| CA08-0072:gltA(K415G) | 30 | 1.7 |

As shown in the results, the K415H variant had an increased L-valine-producing ability without a decrease in growth.

The CA08-0072:gltA(K415H) was named CA08-1688 and deposited at the Korean Culture Center of Microorganisms (KCCM) under Budapest Treaty on September 28, 2020, with Accession No. KCCM12795P.

### Example 3: Construction of O-Acetyl-L-Homoserine Production-Enhancing Strain and Evaluation of O-Acetyl-L-Homoserine-Producing Ability

### 3-1. Construction of Strain Introduced with Foreign Membrane Protein Variant YjeH

In order to determine the effectiveness of the YjeH variant, which is a foreign membrane protein and an O-acetyl homoserine exporter, introduced into *Corynebacterium glutamicum* ATCC13032, a vector for chromosomal introduction containing the gene (SEQ ID NO: 48) encoding the YjeH variant (SEQ ID NO: 47) was constructed.

Specifically, in order to construct a transposase-deleted vector, a primer pair for amplifying the 5' upstream region (SEQ ID NOS: 39 and 40) and a primer pair for amplifying the 3' downstream region (SEQ ID NOS: 41 and 42) around the gene encoding the transposase (SEQ ID NO: 38, gene No. NCgl2335) were designed. The Xbal restriction enzyme site was inserted at each end of the primers of SEQ ID NOS: 39 and 42, and the primers of SEQ ID NOS: 40 and 41 were designed so as to cross each other such that the sequence of the Smal restriction enzyme was located at the designed site. The primer sequences are shown in Table 6.

**[Table 6]**

| SEQ ID NO: | Name of Sequences | Sequences |
|---|---|---|
| SEQ ID NO: 39 | Tn_5 F | tgaattcgagctcggtacccCACCGACGCGCATCTGCCT |
| SEQ ID NO: 40 | Tn_5 R | GGTGTGGTGACTTTCAGCAGTTCCCGGGGGGGA GGAGGCATGTGGTGTTG |
| SEQ ID NO: 41 | Tn_3 F | CAACACCACATGCCTCCTCCCCCCCGGGAACTG CTGAAAGTCACCACACC |
| SEQ ID NO: 42 | Tn_3 R | gtcgactctagaggatccccCTCCCAAACCATTGAGGAAT GG |

PCR was performed using primer pairs of SEQ ID NOS: 39 and 40, and SEQ ID NOS: 41 and 42, based on the chromosome of the wild-type ATCC13032 as a template. The PCR was performed under conditions of denaturation at 95°C for 5 minutes, followed by 30 cycles of denaturation at 95°C for 30 seconds, annealing at 55°C for 30 seconds, and polymerization at 72°C for 30 seconds, and then polymerization at 72°C for 7 minutes. As a result, a DNA fragment (851 bp) in the 5' upstream region and a DNA fragment (847 bp) in the 3' downstream region around the NCgl2335 gene-deleted region were obtained.

PCR was performed using a primer pair of SEQ ID NOS: 39 and 42, based on the two amplified DNA fragments as a template. The PCR was performed under conditions of denaturation at 95°C for 5 minutes, followed by 30 cycles of denaturation at 95°C for 30 seconds, annealing at 55°C for 30 seconds, and polymerization at 72°C for 90 seconds, and then polymerization at 72°C for 7 minutes. As a result, a DNA fragment (1648 bp) including a site capable of deleting the gene encoding the transposase (SEQ ID NO: 38, gene No. NCgl2335) was amplified.

The thus-obtained PCR products were subjected to fusion cloning into the pDCM2 vector treated with the Smal restriction enzyme using an In-Fusion^{®} HD cloning kit (Clontech). The cloned vector was transformed into *E. coli* DH5α, and the transformed *E. coli* was plated on a LB solid medium containing 25 mg/L of kanamycin. Colonies transformed with the plasmid into which the target gene is inserted were selected through PCR, and then the plasmid was obtained by plasmid extraction. Finally, a pDCM2-△NCgl2335 recombinant vector, in which the NCgl2335-deleted cassette is cloned, was constructed.

In order to determine the effectiveness of the O-acetyl homoserine exporter, a vector for chromosomal introduction containing the gene (SEQ ID NO: 48) encoding the YjeH variant derived from *E. coli* was constructed. To this end, a vector expressing the *yjeH* gene was constructed using the CJ7 promoter (US 7662943 B2). A primer pair (SEQ ID NOS: 43 and 44) for amplifying the CJ7 promoter region and a primer pair (SEQ ID NOS: 45 and 46) for amplifying the yjeH region of *E. coli* were designed. The primer sequences are shown in Table 7 below.

**[Table 7]**

| SEQ ID NO: | Name of Sequences | Sequences |
|---|---|---|
| SEQ ID NO: 43 | CJ7_yjeH F | ACACCACATGCCTCCTCcccAGAAACATCCCAG CGCTAC |
| SEQ ID NO: 44 | CJ7_yjeH R | AGTTCTTGTTTGAGTCCACTCATAGTGTTTCCT TTCGTTGGGT |
| SEQ ID NO: 45 | yjeH F | ACCCAACGAAAGGAAACACTATGAGTGGACTC AAACAAGAACTG |
| SEQ ID NO: 46 | yjeH R | GACTTTCAGCAGTTcccgggTTATGTGGTTATGC CATTTTCCGG |

PCR was performed using a primer pair of SEQ ID NOS: 43 and 44, based on the pECCG1 17-PCJ7-gfp(US 7662943 B2, p117-Pcj7-gfp) as a template, and using a primer pair of SEQ ID NOS: 45 and 46, based on the chromosome of the wild-type *E. coli* as a template. The PCR was performed under conditions of denaturation at 95°C for 5 minutes, followed by 30 cycles of denaturation at 95°C for 30 seconds, annealing at 55°C for 30 seconds, and polymerization at 72°C for 90 seconds, and then polymerization at 72°C for 7 minutes. As a result, a DNA fragment (360 bp) of the CJ7 promoter region and a DNA fragment (1297 bp) of the *yjeH* gene region of *E. coli* were obtained.

PCR was performed using a primer pair of SEQ ID NOS: 43 and 46, based on the two amplified DNA fragments as a template. The PCR was performed under conditions of denaturation at 95°C for 5 minutes, followed by 30 cycles of denaturation at 95°C for 30 seconds, annealing at 55°C for 30 seconds, and polymerization at 72°C for 90 seconds, and then polymerization at 72°C for 7 minutes. As a result, a DNA fragment (1614 bp) including the sites where the CJ7 promoter and the *yjeH* gene are introduced was amplified.

The gene-deleted DNA fragment obtained through PCR was cloned into the pDCM2-△NCgl2335 vector treated with the Smal restriction enzyme using an In-Fusion^{®} HD cloning kit (Clontech), and a pOCM2-△NCgl2335::PCJ7-yjeH(eco,WT) recombinant vector was constructed.

Additionally, a recombinant vector for introducing the *yjeH* mutant gene (eco, F351L) was constructed.

Specifically, phenylalanine, which is the 351^{st} amino acid of the YjeH amino acid sequence, was substituted with leucine (F351L) based on the pDCM2-△NCgl2335::PCJ7-yjeH(eco,WT) plasmid as a template using a primer pair of SEQ ID NOS: 49 and 50. The plasmid containing the thus-constructed gene encoding the YjeH(F351L) mutation was named pDCM2-△NCgl2335::PCJ7-yjeH(eco,F351L).

The primer sequences are shown in Table 8 below.

**[Table 8]**

| SEQ ID NO: | Name of Sequences | Sequences |
|---|---|---|
| SEQ ID NO: 49 | F351L F | CAATGGCATCCTTATTATGATTT |
| SEQ ID NO: 50 | F351L R | AAATCATAATAAGGATGCCATTG |

The thus-obtained pDCM2-△NCgl2335 and pDCM2-△NCgl2335::PCJ7-yjeH(eco,F351L) were transformed into the ATCC13032 strain by an electric-pulse method. Through a secondary cross-over, NCgl2335-deleted ATCC13032 △NCgl2335 and ATCC13032 △NCgl2335::PCJ7-yjeH(eco,F351L) were obtained on the chromosome. The deletion of the NCgl2335 gene and the insertion of the gene encoding the YjeH variant were finally confirmed by PCR using a primer pair of SEQ ID NOS: 39 and 42, and then comparing with ATCC13032.

### 3-2. Evaluation of O-Acetyl Homoserine-Producing Ability

In order to compare the O-acetyl homoserine (O-AH)-producing ability of the ATCC13032 △NCgl2335 and ATCC13032 △NCgl2335::PCJ7-yjeH(eco,F351L) strains constructed in Example 3-1, and the wild-type ATCC13032 strain, the strains were cultured in the following manner to analyze O-acetyl homoserine in the medium solution.

One platinum loop of the strains was inoculated into a 250-ml corner-baffled flask containing 25 ml of the O-acetyl homoserine production medium below, and then cultured with shaking at 200 rpm at 33°C for 20 hours. The O-acetyl homoserine concentration was analyzed by HPLC, and the analyzed concentrations are shown in Table 9.

### O-Acetyl Homoserine Production Medium (pH 7.2)

Glucose 30 g, KH₂PO₄ 2 g, Urea 3 g, (NH₄)₂SO₄ 40 g, Peptone 2.5 g, Corn Steep Liquor (CSL, Sigma) 5 g (10 ml), MgSO₄.7H₂O 0.5 g, CaCO₃ 20 g (based on 1 L of distilled water)

**[Table 9]**

| Name of Strains | O-Acetyl Homoserine (g/L) |
|---|---|
| ATCC13032 | 0.3 |
| ATCC13032 △NCgl2335 | 0.3 |
| ATCC13032 △NCgl2335::PCJ7-yjeH(eco,F351L) | 1.0 |

As a result, as shown in Table 9, when the control strain ATCC13032 was cultured, O-acetyl-L-homoserine was accumulated at 0.3 g/L, and it was confirmed that even when the transposase NCgl2335 gene was deleted, it had no effect on the O-acetyl-L-homoserine production. In particular, it was confirmed that when the *yjeH* mutant gene was expressed, the O-acetyl-L-homoserine was accumulated at 1.0 g/L.

### 3-3. Introduction of GltA Variants (K415H) into O-Acetyl-L-Homoserine-Producing Strains and Evaluation thereof

The O-acetyl-L-homoserine-producing ability was evaluated by introducing the GltA variant into the O-acetyl-L-homoserine-producing strains of Example 3-2. The pDCM2-gltA(K415H) vector constructed in Example 1 was transformed into each of the wild-type ATCC13032 and ATCC13032 △NCgl2335 strains, and the O-acetyl-L-homoserine-producing strain of ATCC13032 △NCgl2335::PCJ7-yjeH(eco,F351L). The strains introduced with the vector on the chromosome by recombination of homologous sequences were selected in a medium containing 25 mg/L of kanamycin.

Thereafter, the gene fragments were amplified based on the *Corynebacterium glutamicum* transformants, in which the secondary recombination is completed, by PCR using a primer pair of SEQ ID NOS: 23 and 24, and the strains introduced with the gltA(K415H) mutation were confirmed by sequencing analysis. The recombinant strains were named based on the *Corynebacterium glutamicum* as shown below, and the titer evaluation was carried out in the same manner as in Example 3-2. The results are shown in Table 10.

**[Table 10]**

| Strains | O-AH (g/L) |
|---|---|
| ATCC13032 | 0.3 |
| ATCC13032 gltA(K415H) | 0.4 |
| ATCC13032 △NCgl2335 | 0.3 |
| ATCC13032 △NCgl2335 gltA(K415H) | 0.4 |
| ATCC13032 △NCgl2335::PCJ7-yjeH(eco,F351L) | 1.0 |
| ATCC13032 △NCgl2335::PCJ7-yjeH(eco,F351L) gltA(K415H) | 1.3 |

As can be seen from the results, all of the strains introduced with the GltA K415H variant showed an increase in O-acetyl-L-homoserine-producing ability compared to the parent strain in which the variant is not introduced.

The ATCC13032 △NCgl2335::PCJ7-yjeH(eco,F351L) gltA(K415H) was named CM04-1006 and deposited at the Korean Culture Center of Microorganisms (KCCM) under Budapest Treaty on October 21, 2020, with Accession No. KCCM12809P.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
Corynebacterium glutamicum CA08-1688 | Accession number given by the
INTERNATIONAL DEPOSITARY AUTHORITY:
KCCM12795P

II. SCIENTIFIC DESCRIPTION AND/OR PROPOSED TAXONOMIC DESIGNATION

The microorganism identified under I above was accompanied by:
☐ a scientific description
☐ a proposed taxonomic designation
(Mark with a cross where applicable)

III. RECEIPT AND ACCEPTANCE

This International Depositary Authority accepts the microorganism identified under I above,
which was received by it on September. 28. 2020 (date of the original deposit).¹

IV. RECEIPT OF REQUEST FOR CONVERSION

The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY

Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 28. 2020

¹ Where Rule 6.4(d) applies, such date is the date on which the status of international depositary authority was
acquired.

Form BP/4 (sole page)

한국미생물보존센터 사단법인 한국종균협회
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

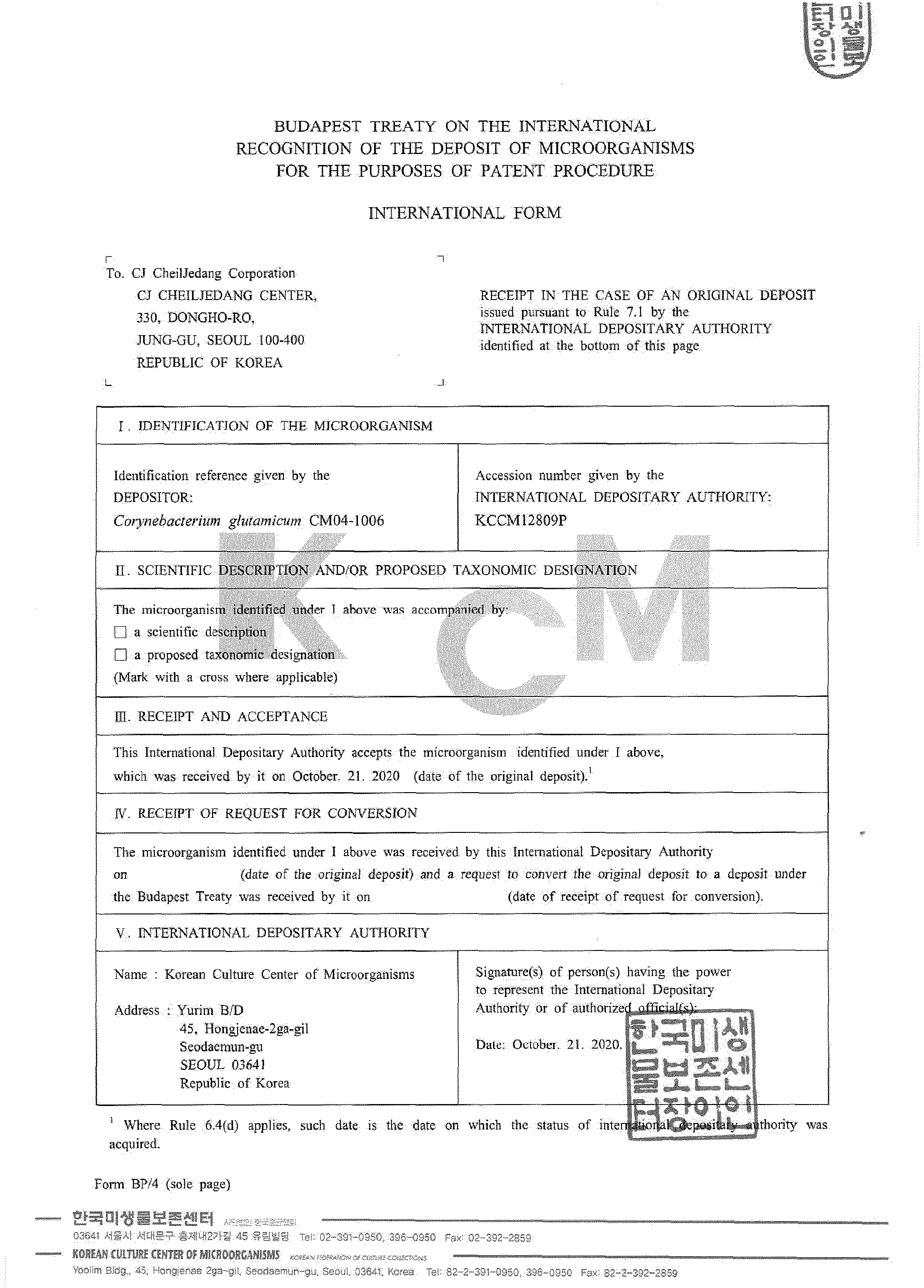

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CM04-1006 | Accession number given by the
INTERNATIONAL DEPOSITARY AUTHORITY:
KCCM12809P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on October. 21. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: October. 21. 2020.
한국미생물보존센터장인

¹ Where Rule 6.4(d) applies, such date is the date on which the status of international depositary authority was
acquired.

Form BP/4 (sole page)

한국미생물보존센터 사단법인 한국종균협회
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A citrate synthase variant in which lysine, which is the amino acid corresponding to position 415 of the amino acid sequence of SEQ ID NO: 1, is substituted with histidine.

2. The variant of claim 1, wherein the variant has a sequence identity of 80% or more with the amino acid sequence of SEQ ID NO: 1.

3. The variant of claim 1, wherein the variant comprises a polypeptide represented by the amino acid sequence of SEQ ID NO: 3.

4. The variant of claim 1, wherein the variant comprises a polypeptide represented by the amino acid sequence of General Formula 1 below:
[General Formula 1] 
X1N HGGDATX2FMN KVKNKEDGVR LMGFGHRVYK NYDPRAAIVK
ETAHEILEHL GGDDLLDLAI KLEEIALADD X3FISRKLYPN VDFYTGLIYR
AMGFPTDFFT VLFAIGRLPG WIAHYREQLG AAGNH (SEQ ID NO: 51);
wherein in General Formula 1 above,
X₁ is asparagine or serine;
X₂ is alanine or glutamic acid; and
X₃ is tyrosine or cysteine.

5. The variant of claim 1, wherein the variant has a sequence identity of 90% or more with the amino acid sequence of SEQ ID NO: 8, 10, or 12.

6. A polynucleotide encoding the variant of any one of claims 1 to 5.

7. A microorganism of the genus *Corynebacterium,* comprising a citrate synthase variant in which lysine, which is the amino acid corresponding to position 415 of the amino acid sequence of SEQ ID NO: 1, is substituted with histidine, or a polynucleotide encoding the variant.

8. The microorganism of claim 7, wherein the microorganism has an L-valine or O-acetyl-L-homoserine-producing ability.

9. The microorganism of claim 7, wherein the microorganism is *Corynebacterium glutamicum.*

10. A method for producing L-amino acids, comprising: culturing a microorganism of the genus *Corynebacterium,* which comprises a citrate synthase variant in which lysine, which is the amino acid corresponding to position 415 of the amino acid sequence of SEQ ID NO: 1, is substituted with histidine, or a polynucleotide encoding the variant, in a medium.

11. The method of claim 10, wherein the method further comprises recovering L-amino acids from the cultured medium or microorganism.

12. The method of claim 10, wherein the L-amino acid is L-valine, O-acetyl-L-homoserine, or L-methionine.

13. A composition for producing L-amino acids, comprising: a microorganism of the genus *Corynebacterium,* which comprises a citrate synthase variant in which lysine, which the amino acid corresponding to position 415 of the amino acid sequence of SEQ ID NO: 1, is substituted with histidine, or a polynucleotide encoding the variant; a medium on which the microorganism is grown; or a combination thereof.

14. The composition of claim 13, wherein the L-amino acid is L-valine, O-acetyl-L-homoserine, or L-methionine.
